# EUROPEAN PATENT SPECIFICATION

(11) **EP 4 346 577 B1**
(45) Date of publication and mention of the grant of the patent: **15.04.2026**
(21) Application number: 22730849.1
(22) Date of filing: 27.05.2022
(51) Int. Cl.: A61B 5/055, A61B 5/11, A61B 5/16, G16H 40/63, G01R 33/28, G16H 20/30, G16H 30/40, G16H 50/20, G01R 33/565, G02B 27/01, G06F 3/01

(54) **PATIENT MOTION SUPPRESSION DURING MEDICAL IMAGING**
PATIENTENBEWEGUNGSUNTERDRÜCKUNG WÄHREND DER MEDIZINISCHEN BILDGEBUNG
SUPPRESSION DE MOUVEMENT DE PATIENT PENDANT L'IMAGERIE MÉDICALE

(30) Priority: 03.06.2021 EP 21177565
(43) Date of publication of application: 10.04.2024
(73) Proprietor: Koninklijke Philips N.V., 5656 AG Eindhoven (NL)
(72) Inventor: WEISS, Steffen, 5656 AG Eindhoven (NL); LEUSSLER, Christoph, Günther, 5656 AG Eindhoven (NL)
(74) Representative: Philips Intellectual Property & Standards
(86) International application number: PCT/EP2022/064402
(87) International publication number: WO 2022/253701

(56) References cited:
- EP-A1- 3 581 109
- CN-A- 110 910 513
- US-A1- 2005 283 068
- US-A1- 2009 010 510
- US-A1- 2011 015 515

## Description

### FIELD OF THE INVENTION

The invention relates to methods and systems for performing patient motion suppression during medical imaging.

### BACKGROUND OF THE INVENTION

Although various schemes for motion correction are applied in clinical magnetic resonance imaging, patient motion is still the most common cause of artefacts. For head scans, ideally, head motion should be limited to below 1mm to allow for image resolution in the order of 1mm. Even motion of the eye balls alone with a stationary head can lead to ghosting artefacts that may obscure anatomical detail in the image.

Motion suppression during medical imaging according to the state of the art is for instance described in EP3581109A1.

### SUMMARY OF THE INVENTION

According to the invention, there is provided a medical imaging method, apparatus and computer-readable medium according to claims 1, 12 and 13, advantageous embodiments being defined in the dependent claims.

### SUMMARY OF THE DISCLOSURE

It would be advantageous/desirable to suppress patient motion during medical imaging. To better address one or more of these concerns, in a first aspect of invention a computer-implemented method for performing motion suppression in a medical imaging apparatus is provided. The method comprises: obtaining image data defining an image to be displayed to a patient within a bore of the medical imaging apparatus; controlling a display device to display the image to the patient in the bore; obtaining data indicating a real-time position of an anatomical region of interest comprising the head and/or eyes of the patient during a medical imaging scan; detecting movement of the anatomical region of interest using the data indicating the real-time position; and in response to detecting the movement, adapting the displayed image to perform patient motion suppression by relocating the displayed image to a location relative to the bore which urges the patient to return their head and/or eyes to their original position..

In one example, the display device comprises a projector configured to project the image onto a display surface in the bore. The display surface may be an inner surface of the bore or a surface provided specifically for the purposes of display. Adapting the image thus comprises controlling the projector to change the region of the display surface e.g. bore wall onto which the image is projected. In another example, the display device comprises a head-mounted display device (HMD) configured to produce a virtual reality display or augmented reality display, wherein adapting the image comprises changing the apparent location of the (virtual) image, which in an AR scenario may be world-locked to a part of a region of the bore wall designated as a display surface. It will be apparent from the above that relocating the image may thus comprise relocating the boundary or frame of the image and/or its center, as opposed to moving content within the image but without moving the boundary, frame, or center of the image.

The location may be a real location when the image is projected onto the display surface in the bore or a virtual location in the case of VR/AR. In other words, the patient is urged to move their head and/or eyes so as to reverse the detected movement of the anatomical region of interest. By "head and/or eyes" is meant that motion of the image is relative to one or both of head movement and eye movement of the patient. In one example, motion of the image is relative to the gaze direction of the patient. In that example, the image may be moved in response to head movement even if the gaze direction remains unchanged.

In one example of performing patient motion suppression, referring to herein as negative compensation, the method further comprises, in response to detecting movement of the head and/or eyes of the patient towards one side, moving the image towards the opposite side. In this way, the image is adapted to intuitively guide the patient to limit their own head motion and eye motion and to return to an original position after any motion.

In a further example of performing patient motion suppression, referring to herein as positive overcompensation, the method further comprises, in response to detecting movement of the head and/or eyes of the patient to one side, moving the image to the same side, wherein the movement of the image is amplified relative to the movement of the head and/or eyes by an amplification factor k. Again, the image is adapted to intuitively guide the patient to limit their own head motion and eye motion and to return to an original position after any motion. The amplification factor relates the magnitude of movement of the head and/or eyes to that of the image. In one example, the amplification factor k may relate an angle of rotation of the projector relative to an angle of rotation of the head and/or eyes (gaze direction). Additionally or alternatively, the amplification factor may relate a lateral displacement in the location of the image relative to a lateral displacement of the patient's point of gaze on the bore wall.

In any of the approaches described herein, the location of the image may be continually adjusted in response to the changing position of the head and/or eyes of the patient. In particular, the method may further comprise moving the image back towards to its original position in response to detecting movement of the head and/or eyes of the patient back towards their original position.

In yet another example of performing patient motion suppression, adapting the image comprises depicting the detected movement of the anatomical region of interest in the image. In this case, the "image" may be understood as a series of images such as video images. Seeing the movement so depicted and being aware of its consequences, the patient is discouraged from moving further and encouraged to return to the their original position. As in other approaches, the detected movement of the anatomical region of interest may be depicted in the image with an amplification factor k. To provide for a body part-specific amplification factor, the method may further comprise determining the amplification factor based at least partly on the anatomical region of interest. Additionally or alternatively, so as to provide a scan-specific amplification factor, the method may further comprise determining the amplification factor based at least partly on a motion sensitivity of the medical imaging scan being performed. Thus, in any of the approaches described herein, the amplification factor may be determined as a function of one or more parameters including, among others, anatomy and scan type. In this way, the use of different amplification factors for different anatomical regions and scan types is enabled, with the amplification factor being adapted to those parameters, so as to provide a context-aware form of patient motion suppression in which the suppressive effect is strengthened when it is most needed and relaxed at other times.

The image may comprise a monoscopic image (with one image directed to both of the patient's eyes) or a stereoscopic image (with two distinct images directed individually to each of the patient's eyes for a 3D effect). In either case, when the detected movement of the anatomical region of interest is depicted in the image, the anatomical region of interest may be depicted in three dimensions. In this way, the patient is better able to discern movement of the anatomical region of the interest in the image.

In examples in which the image is displayed by being projected, the method may comprise correcting for primary and/or secondary distortion. In particular, the method may comprise applying one or more transformations to the image data to compensate for the primary and/or secondary distortion. To correct for primary distortion, the method may comprise applying one or more transformations to the image data to correct for skew distortion caused by an off-center position of the projector and/or for curvature of the inner surface of the bore. To correct for secondary image distortion, the method may further comprise using the data indicating the real-time position to detect an off-center viewing position of the patient, and applying a perspective transformation to the image data to adapt the projected image to the off-center viewing position. Stated differently, correcting for secondary image distortion may comprise: determining, based on a current position of a patient within the bore and on a current projection direction of the projector, whether the projected image will appear distorted to the patient owing to an off-center viewing perspective of the patient; determining a perspective transformation that will transform the image data to correct for the off-center viewing position; and transforming the image data according to the determined perspective transformation. Primary and/or secondary distortion correction may be performed only when it is determined from the gaze direction that the patient is looking directly at the projected image. Appropriate transformations for the distortion correction may be determined using data defining the display surface, particularly where this is formed by the inner surface of the bore. Predetermined transformations may be selected on the basis of the data defining the current patient position and the current projection direction, for example using one or more lookup tables to relate the patient position and projector direction to a particular transformation.
The data indicating the real-time position of the anatomical region of interest may be obtained using a patient positioning system, for example a camera-based positioning system and/or using the medical imaging apparatus itself. Patient positioning may comprise gaze tracking to determine a current eye position and/or gaze direction and/or gaze point of the patient. Detecting movement of the anatomical region of interest may comprise detecting movement above a threshold distance, velocity, or acceleration using the data indicating the real-time position. Detecting movement of the anatomical region of interest may further comprise detecting movement via a sensor or marker attached to the patient, for example a motion sensor, marker, RF coil (e.g. on the body), or a helmet coil.

In a restatement of the first aspect, there is provided a computer-implemented method for performing motion suppression in a medical imaging apparatus. The method comprises: obtaining image data defining an image to be displayed to a patient within a bore of the medical imaging apparatus; controlling a display device to display the image to the patient in the bore; obtaining data indicating a real-time position of an anatomical region of interest during a medical imaging scan; detecting movement of the anatomical region of interest using the data indicating the real-time position; and in response to detecting the movement, adapting the displayed image to perform patient motion suppression. Adapting the image to perform patient motion suppression may in this case comprise changing the location at which the image is displayed and/or changing the image content itself, for example changing the image content to depict movement of the anatomical region of interest. The anatomical region of interest may comprise any part of the patient's anatomy, including for example a leg or an arm.

According to a second aspect, there is provided a method of correcting for secondary distortion during in-bore projection in a medical imaging apparatus. The method comprises: obtaining image data defining an image to be displayed to a patient within a bore of the medical imaging apparatus; obtaining data indicating a real-time position of the patient within the bore during a medical imaging scan; using the data indicating the real-time position to detect an off-center viewing position of the patient; applying a perspective transformation to the image data to adapt the image to the off-center viewing position; and controlling the projector to project the image onto a display surface in the bore using the transformed image data. The term "controlling" as used herein in relation to the projector may refer to controlling the image content which is output by the projector and/or controlling the position and/or projection direction of the projector.

According to a third aspect, there is provided a method of controlling a medical imaging apparatus. The method comprises: controlling the medical imaging apparatus to perform a medical imaging scan for imaging an anatomical region of interest of a patient; and during the medical imaging scan, performing the method of the first and/or second aspect.

According to a fourth aspect, there is provided a controller configured to perform the method of any of the first-third aspects. The controller may comprise a computing device comprising a processor configured to perform the method of any of the first-third aspects.

According to a fifth aspect, there is provided a medical imaging apparatus comprising the controller of the fourth aspect.

According to a sixth aspect, there is provided a computer program product comprising instructions which, when executed by a computing device, enable or cause the computing device to perform the method of any of the first-third aspects.

According to a seventh aspect, there is provided a computer-readable medium comprising instructions which, when executed by a computing device, enable or cause the computing device to perform the method of any of the first-third aspects.

Thus provided by the present disclosure is a form of motion-suppressing in-bore projection display based on a modified distortion correction. Any optional feature or sub-aspect of one of the above aspects applies as appropriate to any of the other aspects.

These and other aspects will be apparent from and elucidated with reference to the embodiments described hereinafter.

### BRIEF DESCRIPTION OF THE DRAWINGS

A detailed description will now be given, by way of example only, with reference to the accompanying drawings, in which:
Figs. 1-3 illustrate an exemplary medical imaging apparatus; and
Fig. 4 illustrates a computing device that can be used in accordance with the systems and methods disclosed herein.

### DETAILED DESCRIPTION OF EMBODIMENTS

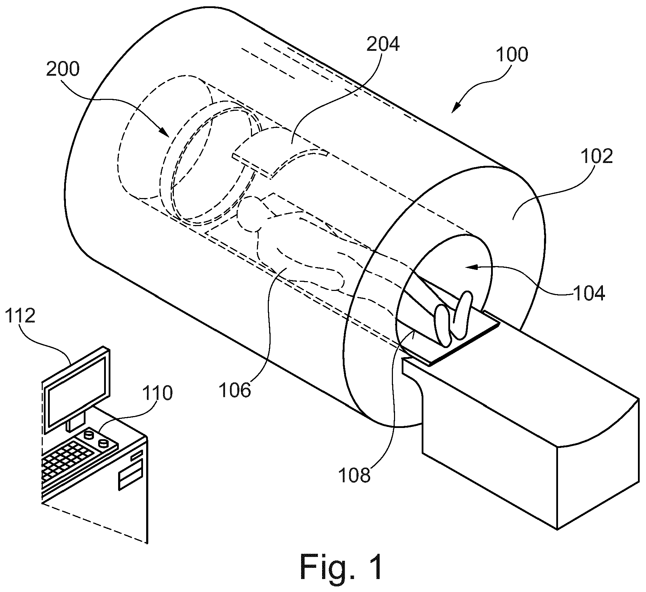

Fig. 1 illustrates an exemplary medical imaging apparatus 100, which in this example is a magnetic resonance imaging (MRI) apparatus. The MRI apparatus 100 comprises a housing 102 containing typical components of an MRI apparatus such as a magnet, magnetic field gradient coils used to create magnetic spins within an imaging zone, and a radio-frequency transceiver for manipulating the orientations of the magnetic spins within the imaging zone and for receiving radio transmissions from the imaging zone. A bore 104 is formed through the housing 102 in which a patient 106 may be positioned for imaging by a patient support 108 such that the imaging zone contains an anatomical region of interest. The MRI apparatus 100 comprises a computer-implemented control system 110 comprising a user interface 112. The control system 110 controls the operation and function of the MRI apparatus 100. In particular, the control system 110 controls the MRI apparatus 100 to perform an MRI scan according to a particular scan sequence. Individual scans in the sequence may comprise for example a gradient echo scan, an echo-planar scan, a spin-echo scan, or a diffusion scan, as is known in the art. The acquired magnetic resonance data be used to reconstruct one or more magnetic resonance images.

An in-bore communication system 200 is provided for providing in-bore patient communication. The in-bore communication system 200 may provide for audio communication between the patient 106 in the bore 104 and an MR technician at the user interface 112. The in-bore communication system 200 may also comprise an emergency button to allow the patient 106 to call for attention during MR scanning, during which audio communication may be switched off. In particular, the in-bore communication system 200 comprises an in-bore projector for projecting an image 204 onto an inner surface of the bore 104. The image 204 may be a passive image or the projection may be used to provide the patient 106 with a graphical user interface similar to a touch screen. The projected image 104 may comprise any one or more of image content, text content, and interactive soft keys.

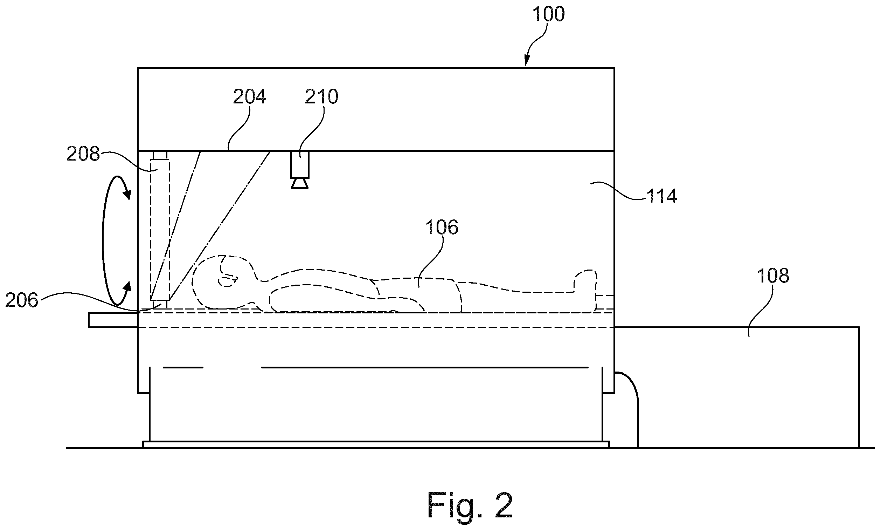

Fig. 2 illustrates the in-bore projector 206 being used to project the image 204 onto the inner surface 114 of the bore 104. The in-bore projector 206 is movably affixed to an annular gantry 208 so as to be rotatable around the periphery of the bore 104 in order to project the image 204 onto different regions of the inner surface 114 of the bore 104. In particular, the in-bore projector 206 may be moved to a position from which it projects the image 204 onto a region of the inner surface 114 which coincides with a detected gaze direction of the patient 106. A patient positioning system 210 located within the bore 104 may be used to detect the current position of the patient 106, in particular the current gaze direction of the patient 106. In the example shown, the patient positioning system 210 comprises a camera.

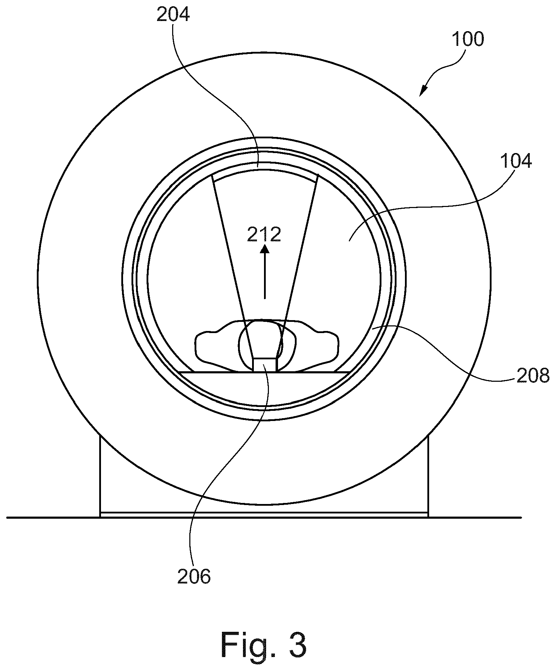

Fig. 3 shows the detected gaze direction 212 of the patient 106 being used to position the in-bore projector 206 on the gantry 208 so as to project the image 204 onto a region of the inner surface 114 of the bore 104 which coincides with the detected gaze direction 212.

It will be apparent from Figs. 2 and 3 that, if left uncorrected, image distortion will occur due to the curvature of the inner surface 114 of the bore 104 and the off-center position of projector 206. By "off-center projection" or "off-axis projection" is meant herein that the in-bore projector 206 is not located on an axis that extends perpendicularly from the center of the projected image 204 or, stated differently, that the display surface does not reside in a plane which is orthogonal to the optical axis of the projector, as is evident from Fig. 2. In this case, projecting rectangular content would produce a trapezoid on a flat inner surface 114 due to the distortion caused by the off-axis projection. Image distortion resulting from off-center projection and bore wall curvature is referred to herein as primary distortion. The control system 110 is configured to correct for the primary distortion by transforming the image data in software using one or more transformations such as a trapezoid correction, thereby allowing a rectangular image 204 to be projected onto the curved inner surface 114 of the bore 104 during off-axis projection. Known techniques such as 3D projection mapping may be used to correct for the primary distortion.

In addition to the primary distortion, the present disclosure envisages a form of secondary distortion caused by an off-axis eye position of the patient. An off-center or off-axis eye position occurs when the patient's gaze direction 212 does not coincide with the axis that extends perpendicularly from the center of the projected image 204 or, stated differently, when the display surface does not reside in a plane which is orthogonal to the patient's gaze direction. The secondary distortion results from the fact that, when the patient 106 observes the image 204 from such an off-axis eye position, a projected image 204 that is actually rectangular on the bore wall is perceived to be non-rectangular. The control system 110 is furthermore configured to correct for the secondary distortion by transforming the image data so that the patient 106 sees rectangular image content without any distortion as if the patient's head were at the optimal position with respect to the projected image 204. The patient positioning system 210 is used to measure the position of the eyes and the gaze direction of the patient 106. The secondary correction to be applied may be defined as the inverse of the (fictive) image transform that would be applied for primary correction if the projector and the eyepoint were at identical off-axis positions. Known dynamic anamorphic image projection methods may be used to correct for the secondary distortion, such as that described in "Dynamic anamorphosis", Franc Solina et al, Proceedings of ENACTIVE/07, 4th International Conference on Enactive Interfaces, Grenoble, France, November 19-22, 2007.

In some applications, the control system 110 may be configured to control the in-bore projector 206 to project the image 204 onto a region of the inner surface 114 which coincides with the detected gaze direction of the patient 106. Thus, if the patient 106 turns their head to the left or moves their eyes to the left, the projected image 204 on the bore wall is also moved to the left. Simultaneously, the image data is corrected for primary and secondary distortion in the above-described manner so as to morph the image 204 such that, even when viewed from the new perspective, the patient 106 still sees rectangular content on the curved bore wall. Instead of simply tracking the gaze direction, the present disclosure proposes to relocate the image in such a way as to limit patient head and eye motion for better quality medical imaging. In particular, the projected image 204 is further modified to intuitively guide the patient 106 to limit their own head motion and eye motion and to return to an original position after any motion. Multiple ways of performing patent motion suppression will now be described.

A first approach to performing patient motion suppression is referred to herein as negative compensation. Using this approach, if the patient 106 turns their head to the left, the projected image 204 on the bore wall is moved to the right, and vice versa. Equally, if the patient moves their gaze direction down, the image is moved up. Thus, when the patient 106 starts moving their head or gaze direction, they lose sight of the image 204 more quickly than if the image 204 were static. This urges the patient 106 to return to the original position, effectively suppressing patient motion and thereby improving image quality. Patient motion, in particular changes in gaze direction, as detected by the patient positioning system 210 is communicated to the control system 110, which controls the in-bore projector 206 to move the image 204 in a manner according to the negative compensation approach, i.e. in the opposite direction to that in which the gaze direction moves, while optionally also adapting the image data to correct for primary and/or secondary image distortion.

A second approach to performing patient motion suppression is referred to herein as positive overcompensation. Using this approach, if the patient 106 moves their gaze direction in one direction (e.g. to the left), the projected image 204 on the bore wall is moved in the same direction (e.g., to the left) but by an amplification factor k relative to the movement that would be required for the image 204 simply to track the changing gaze direction 212. The patient 106 thus receives feedback on their motion with some exaggeration by strong motion of the projected image 204. Again, the patient 106 begins to lose sight of the image 204 as a result of their motion, which effectively suppresses the patient motion, thereby improving image quality.

Different scans have different sensitivity to motion. The present disclosure further proposes to increase the amplification factor k according to scan motion sensitivity. Advantageously, the patient motion suppression effect is thereby stronger for more motion-sensitive scans.

A further approach to performing patient motion suppression comprises adapting the image content to depict detected movement of an anatomical region of interest in the projected image itself. This is particularly effective when the detected movement of the anatomical region of interest is depicted in the image with an amplification factor k, and when the image is a stereographic 3D image, depicting e.g. a simplified model of the body of the patient. Stereographic images are more realistic and provide a 3D viewing experience which can be combined with the depiction of the patient motion. Any known technique for realizing a 3D display may be used, including for example stereographic techniques using glasses or head-mounted displays. The amplification factor k is used to define a 3D vector for each pixel. Thus, wherein the anatomical region of interest comprises the head and/or eyes, as in the two approaches described above, movement of the head and/or eyes may be depicted in the image to discourage the patient from moving. This approach is applicable also to other body parts, however, with the depicted body part establishing a ground truth concerning patient motion. Additionally, particularly in a 3D display, the imaging slice can be made visible in the image. Using this approach, the head may be located on a head rest, as the 3D head motion is visualized and the head can be repositioned exactly.

In one use case, the image may be adapted to provide guidance for cinematic studies in which the head (or other anatomical region of interest) needs to be moved by rotating it to the left or right or by nodding. Medical imaging is performed continuously or in discrete steps. The patient's head is depicted in the image alongside a visualization of the required position to assist the patient in maintaining the head in the currently required position or rapidly moving the head into the next required position. The visualization may comprise a symbolic depiction of a head in the required position. The visualization may comprise a sequence of required positions for the patient to follow, synchronized in time with an MRI sequence. Patient motion suppression using an amplification factor may be performed, as described elsewhere herein.

In a further use case, medical imaging is performed at multiple head orientations with respect to a magnetic field for quantitative MRI (T2* and magnetic susceptibility). In this case, the head needs to be located and locked at a different angle for the duration of a sequence. Uncomfortable positions may not be held by the patient for the required duration and may therefore be repeated.

In a yet further use case, a monoscopic VR display renders an image of the head against a reference grid, so that the patient can follow a defined motion path (e.g. in one plane corresponding to a nodding motion). Deviation from the optimal motion path is displayed with respect to the reference plane. When motion along the motion path has been completed, the display switches to a different plane (e.g. a second plane corresponding to movement of the head to the left and right). Stereoscopic VR can be applied for more complex movement such as free rotation (both 'nodding' and movement to the left and right) of an anatomical region of interest such as the head, neck, knee, hips, or other joint to find a point or region of pain. Here, a 3D reference grid may be displayed to assist the patient in visualizing the required motion path of the anatomical region of interest. The anatomical region of interest may be depicted symbolically (using e.g. lines, connection-points) with respect to the 3D reference grid. The motion of the anatomical region of interest may be tracked and stored to allow the same motion to be repeated to better define the region of pain.

In any of the above-described approaches, the amplification factor may be determined based at least partly on the anatomical region of interest. Individual sources of motion i may be amplified with a different amplification factor *ki* when depicted in the projected image. The depiction may be symbolic, using for example symbols, pictograms, animation, and so on. Sources of motion include: tongue position associated with amplification factor *kl*; facial expression with factor *k2*; eye movement with factor *k3*; and/or head movement with factor *k4.* For example, head movement and tongue movement can be simultaneously depicted in the projected image. Individual motion is detected by different sensors, for example infrasound or haptic systems or additional cameras placed e.g. in a head coil to provide higher resolution for face and tongue tracking.

It will be understood that the details of the MRI apparatus are provided herein for the purposes of illustration only and that the techniques described herein may be applied to any form of medical imaging which is subject to motion artefacts, including for example computed tomography scans.

Although the methods are described herein as being performed by control system 110 of the MRI apparatus 100, it will be appreciated that the methods could equally be performed by a dedicated controller, by the controller of another component (such as that of the in-bore communication system or projector), or by multiple controllers together forming a distributed control system.

Although a separate camera is shown for implementing the patient positioning system, it will be appreciated that other forms of patient positioning may be used or that the medical imaging apparatus itself may be used to perform patient positioning.

The projector described above is located inside the bore but it will be understood that the projector may be located outside of the bore while still being configured to project images onto the display surface. In other arrangements, non-projection based display devices may replace the projector, such as a head-mounted display device providing a virtual reality display or augmented reality display.

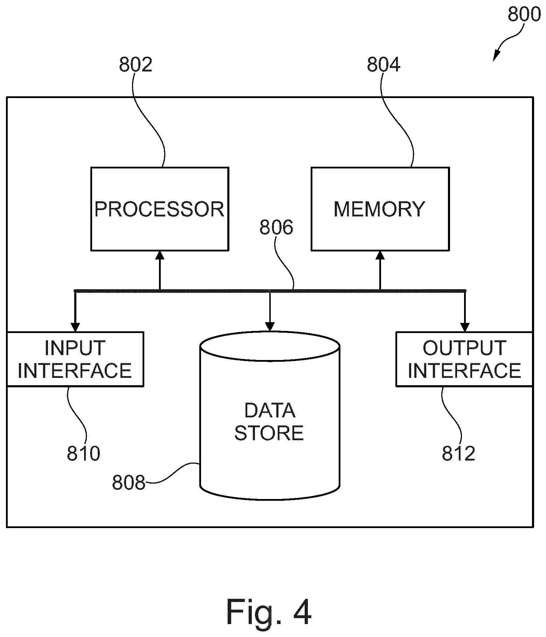

Referring now to Fig. 4, a high-level illustration of an exemplary computing device 800 that can be used in accordance with the systems and methodologies disclosed herein is illustrated. The computing device 800 includes at least one processor 802 that executes instructions that are stored in a memory 804. The instructions may be, for instance, instructions for implementing functionality described as being carried out by one or more components discussed above or instructions for implementing one or more of the methods described above. The processor 802 may access the memory 804 by way of a system bus 806. In addition to storing executable instructions, the memory 804 may also store conversational inputs, scores assigned to the conversational inputs, etc.

The computing device 800 additionally includes a data store 808 that is accessible by the processor 802 by way of the system bus 806. The data store 808 may include executable instructions, log data, etc. The computing device 800 also includes an input interface 810 that allows external devices to communicate with the computing device 800. For instance, the input interface 810 may be used to receive instructions from an external computer device, from a user, etc. The computing device 800 also includes an output interface 812 that interfaces the computing device 800 with one or more external devices. For example, the computing device 800 may display text, images, etc. by way of the output interface 812.

It is contemplated that the external devices that communicate with the computing device 800 via the input interface 810 and the output interface 812 can be included in an environment that provides substantially any type of user interface with which a user can interact. Examples of user interface types include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable a user to interact with the computing device 800 in a manner free from constraints imposed by input device such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth.

Additionally, while illustrated as a single system, it is to be understood that the computing device 800 may be a distributed system. Thus, for instance, several devices may be in communication by way of a network connection and may collectively perform tasks described as being performed by the computing device 800.

Various functions described herein can be implemented in hardware, software, or any combination thereof. If implemented in software, the functions can be stored on or transmitted over as one or more instructions or code on a computer-readable medium. Computer-readable media include computer-readable storage media. Computer-readable storage media can be any available storage media that can be accessed by a computer. By way of example, and not limitation, such computer-readable storage media can comprise FLASH storage media, RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal is not included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), etc.

It will be appreciated that the aforementioned circuitry may have other functions in addition to the mentioned functions, and that these functions may be performed by the same circuit.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein.

It has to be noted that embodiments are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the disclosure has been illustrated and described in detail in the drawings and foregoing description, such illustration and description are to be considered exemplary and not restrictive. The disclosure is not limited to the disclosed embodiments. Other variations to the disclosed embodiments can be understood and effected by those skilled in the art, from a study of the drawings, the disclosure, and the appended claims.

The word "comprising" does not exclude other elements or steps.

The indefinite article "a" or "an" does not exclude a plurality. In addition, the articles "a" and "an" as used herein should generally be construed to mean "one or more" unless specified otherwise or clear from the context to be directed to a singular form.

A single processor or other unit may fulfil the functions of several items recited in the claims.

The mere fact that certain measures are recited in mutually different claims does not indicate that a combination of these measures cannot be used advantageously.

A computer program may be stored/distributed on a suitable medium, such as an optical storage medium or a solid-state medium supplied together with or as part of other hardware, but may also be distributed in other forms, such as via the internet or other wired or wireless communications systems.

Any reference signs in the claims should not be construed as limiting the scope.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "X comprises A and/or B" is satisfied by any of the following instances: X comprises A; X comprises B; or X comprises both A and B.

## Claims

1. A computer-implemented method for performing motion suppression in a medical imaging apparatus (100), the method comprising:
- obtaining image data defining an image (204) to be displayed to a patient (106) within a bore (104) of the medical imaging apparatus;
- controlling a display device (206) to display the image to the patient in the bore;
- obtaining data indicating a real-time position of an anatomical region of interest comprising the head and/or eyes of the patient during a medical imaging scan;
- detecting movement of the anatomical region of interest using the data indicating the real-time position; and
- in response to detecting the movement, adapting the displayed image to perform patient motion suppression by relocating the displayed image to a location relative to the bore which urges the patient to return their head and/or eyes to their original position, **characterised in that** relocating the image comprises relocating a frame of the image.

2. The method of claim 1, further comprising, in response to detecting movement of the head and/or eyes of the patient towards one side, moving the displayed image towards the opposite side.

3. The method of claim 1, further comprising, in response to detecting movement of the head and/or eyes of the patient to one side, moving the displayed image to the same side, wherein the movement of the image is amplified relative to the movement of the head and/or eyes by an amplification factor k.

4. The method of any preceding claim, further comprising moving the displayed image back towards to its original position in response to detecting movement of the head and/or eyes of the patient back towards their original position.

5. The method of any preceding claim, wherein adapting the displayed image comprises depicting the detected movement of the anatomical region of interest in the displayed image.

6. The method of claim 5, wherein the detected movement of the anatomical region of interest is depicted in the displayed image with an amplification factor k.

7. The method of any preceding claim, further comprising controlling the display device to render the image as a stereographic image.

8. The method of claim 3 or 6, further comprising determining the amplification factor based at least partly on the anatomical region of interest.

9. The method of claim 3, 6 or 8, further comprising determining the amplification factor based at least partly on a motion sensitivity of the medical imaging scan.

10. The method of any preceding claim, further comprising using the data indicating the real-time position to detect an off-center viewing position of the patient, and applying a perspective transformation to the image data to adapt the displayed image to the off-center viewing position.

11. A method of controlling a medical imaging apparatus, the method comprising:
- controlling the medical imaging apparatus to perform a medical imaging scan for imaging an anatomical region of interest of a patient; and
- during the medical imaging scan, performing the motion suppression method of any preceding claim.

12. A medical imaging apparatus (100) having a bore (104), a display device (206) configured to display an image (204) to a patient (106) in the bore and a controller configured to perform the method of any preceding claim.

13. A computer-readable medium comprising instructions which, when executed by a controller of a medical imaging apparatus (100) according to the preceding claim, cause the controller to perform the method of any of claims 1-11.

## Patentansprüche

1. Computerimplementiertes Verfahren zum Durchführen einer Bewegungsunterdrückung in einer medizinischen Bildgebungseinrichtung (100), wobei das Verfahren Folgendes umfasst:
- Gewinnen von Bilddaten, die ein Bild (204) definieren, das einem Patienten (106) innerhalb einer Öffnung (104) der medizinischen Bildgebungseinrichtung angezeigt werden soll;
- Steuern einer Anzeigevorrichtung (206) zum Anzeigen des Bildes für den Patienten in der Öffnung;
- Gewinnen von Daten, die eine Echtzeitposition eines anatomischen Bereichs von Interesse, der den Kopfes und/oder die Augen des Patienten umfasst, während einer medizinischen Bildgebungsuntersuchung angeben;
- Erkennen einer Bewegung des anatomischen Bereichs von Interesse unter Verwendung der Daten, welche die Echtzeitposition angeben; und
- als Reaktion auf das Erkennen der Bewegung Anpassen des angezeigten Bildes, um eine Unterdrückung der Patientenbewegung durchzuführen, durch Neupositionieren des angezeigten Bildes an eine Position relativ zur Öffnung, die den Patienten nötigt, den Kopf und/oder die Augen an ihre ursprüngliche Position zurückbringen, **dadurch gekennzeichnet, dass** das Neupositionieren des Bildes das Neupositionieren eines Rahmens des Bildes umfasst.

2. Verfahren nach Anspruch 1, ferner umfassend, als Reaktion auf das Erkennen einer Bewegung des Kopfes und/oder der Augen des Patienten hin zu einer Seite, das Bewegen des angezeigten Bildes zur entgegengesetzten Seite.

3. Verfahren nach Anspruch 1, ferner umfassend, als Reaktion auf das Erkennen einer Bewegung des Kopfes und/oder der Augen des Patienten zu einer Seite, das Bewegen des angezeigten Bildes zu derselben Seite, wobei die Bewegung des Bildes im Verhältnis zur Bewegung des Kopfes und/oder der Augen um einen Verstärkungsfaktor k verstärkt wird.

4. Verfahren nach einem vorstehenden Anspruch, ferner umfassend das Zurückbewegen des angezeigten Bildes hin zu seiner ursprünglichen Position als Reaktion auf das Erkennen einer Bewegung des Kopfes und/oder der Augen des Patienten hin zu ihrer ursprünglichen Position.

5. Verfahren nach einem vorstehenden Anspruch, wobei das Anpassen des angezeigten Bildes das Darstellen der erkannten Bewegung des anatomischen Bereichs von Interesse im angezeigten Bild umfasst.

6. Verfahren nach Anspruch 5, wobei die erkannte Bewegung des anatomischen Bereichs von Interesse im angezeigten Bild mit einem Verstärkungsfaktor k dargestellt wird.

7. Verfahren nach einem vorstehenden Anspruch, ferner umfassend das Steuern der Anzeigevorrichtung zum Wiedergeben des Bildes als stereographisches Bild.

8. Verfahren nach Anspruch 3 oder 6, ferner umfassend das Bestimmen des Verstärkungsfaktors zumindest teilweise auf der Grundlage des anatomischen Bereichs von Interesse.

9. Verfahren nach Anspruch 3, 6 oder 8, ferner umfassend das Bestimmen des Verstärkungsfaktors zumindest teilweise auf der Grundlage einer Bewegungsempfindlichkeit der medizinischen Bildgebungsabtastung.

10. Verfahren nach einem vorstehenden Anspruch, ferner umfassend das Verwenden der Daten, welche die Echtzeitposition angeben, um eine außermittige Betrachtungsposition des Patienten zu erkennen, und das Anwenden einer Perspektivtransformation auf die Bilddaten, um das angezeigte Bild an die außermittige Betrachtungsposition anzupassen.

11. Verfahren zum Steuern einer medizinischen Bildgebungseinrichtung, wobei das Verfahren Folgendes umfasst:
- Steuern der medizinischen Bildgebungseinrichtung zum Durchführen einer medizinischen Bildgebungsabtastung zum Darstellen eines anatomischen Bereichs von Interesse eines Patienten und,
- während der medizinischen Bildgebungsabtastung, Durchführen des Bewegungsunterdrückungsverfahrens nach einem vorstehenden Anspruch.

12. Medizinische Bildgebungseinrichtung (100), die eine Öffnung (104), eine Anzeigevorrichtung (206), die so konfiguriert ist, dass sie einem Patienten (106) in der Öffnung ein Bild (204) anzeigt, und eine Steuereinheit, die so konfiguriert ist, dass sie das Verfahren nach einem vorstehenden Anspruch durchführt, aufweist.

13. Computerlesbares Medium, das Anweisungen umfasst, die, wenn sie von einer Steuereinheit einer medizinischen Bildgebungseinrichtung (100) nach dem vorstehenden Anspruch ausgeführt werden, die Steuereinheit veranlassen, das Verfahren nach einem der Ansprüche 1 bis 11 durchzuführen.

## Revendications

1. Procédé mis en œuvre par ordinateur pour réaliser une suppression de mouvement dans un appareil d'imagerie médicale (100), le procédé comprenant :
- l'obtention de données d'image définissant une image (204) à afficher à un patient (106) dans un alésage (104) de l'appareil d'imagerie médicale ;
- la commande d'un dispositif d'affichage (206) pour afficher l'image au patient dans l'alésage ;
- l'obtention de données indiquant une position en temps réel d'une région anatomique d'intérêt comprenant la tête et/ou les yeux du patient pendant un examen d'imagerie médicale ;
- la détection de mouvement de la région anatomique d'intérêt en utilisant les données indiquant la position en temps réel ; et
- en réponse à la détection du mouvement, l'adaptation de l'image affichée pour réaliser une suppression de mouvement du patient en repositionnant l'image affichée vers un emplacement relatif à l'alésage qui incite le patient à remettre la tête et/ou les yeux dans leur position originale, **caractérisé en ce que** le repositionnement de l'image comprend le repositionnement d'une trame de l'image.

2. Procédé selon la revendication 1 comprenant en outre, en réponse à la détection de mouvement de la tête et/ou des yeux du patient vers un côté, le déplacement de l'image affichée vers le côté opposé.

3. Procédé selon la revendication 1 comprenant en outre, en réponse à la détection de mouvement de la tête et/ou des yeux du patient vers un côté, le déplacement de l'image affichée vers le même côté, dans lequel le mouvement de l'image est amplifié par rapport au mouvement de la tête et/ou des yeux par un facteur d'amplification k.

4. Procédé selon l'une quelconque des revendications précédentes, comprenant en outre le redéplacement de l'image affichée vers sa position originale en réponse à la détection de mouvement de la tête et/ou des yeux du patient vers leur position originale.

5. Procédé selon l'une quelconque des revendications précédentes, dans lequel l'adaptation de l'image affichée comprend la représentation du mouvement détecté de la région anatomique d'intérêt dans l'image affichée.

6. Procédé selon la revendication 5, dans lequel le mouvement détecté de la région anatomique d'intérêt est représenté dans l'image affichée avec un facteur d'amplification k.

7. Procédé selon l'une quelconque des revendications précédentes, comprenant en outre la commande du dispositif d'affichage pour restituer l'image comme une image stéréographique.

8. Procédé selon la revendication 3 ou 6, comprenant en outre la détermination du facteur d'amplification au moins partiellement sur la base de la région anatomique d'intérêt.

9. Procédé selon la revendication 3, 6 ou 8, comprenant en outre la détermination du facteur d'amplification au moins partiellement sur la base d'une sensibilité au mouvement de l'examen d'imagerie médicale.

10. Procédé selon l'une quelconque des revendications précédentes, comprenant en outre l'utilisation des données indiquant la position en temps réel pour détecter une position de visualisation décentrée du patient, et l'application d'une transformation de perspective aux données d'image pour adapter l'image affichée à la position de visualisation décentrée.

11. Procédé de commande d'un appareil d'imagerie médicale, le procédé comprenant :
- la commande de l'appareil d'imagerie médicale pour réaliser un examen d'imagerie médicale pour imager une région anatomique d'intérêt d'un patient ; et
- pendant l'examen d'imagerie médicale, la réalisation du procédé de suppression de mouvement selon l'une quelconque des revendications précédentes.

12. Appareil d'imagerie médicale (100) présentant un alésage (104), un dispositif d'affichage (206) configuré pour afficher une image (204) à un patient (106) dans l'alésage et un dispositif de commande configuré pour réaliser le procédé selon l'une quelconque des revendications précédentes.

13. Support lisible par ordinateur comprenant des instructions qui, lorsqu'elles sont exécutées par un dispositif de commande d'un appareil d'imagerie médicale (100) selon la revendication précédente, amènent le dispositif de commande à réaliser le procédé selon l'une quelconque des revendications 1-11.
